# EUROPEAN PATENT SPECIFICATION

(11) **EP 3 466 542 B1**
(45) Date of publication and mention of the grant of the patent: **30.03.2022**
(21) Application number: 16904738.8
(22) Date of filing: 18.10.2016
(51) Int. Cl.: B01L 3/00, A61M 1/36, C12M 1/26, C12M 1/00, C12N 5/0775, C12N 5/071, B04B 5/04, B04B 7/08, B04B 11/02

(54) **VESSEL FOR CENTRIFUGE, AND SVF ISOLATION METHOD USING SAME**
BEHÄLTER FÜR ZENTRIFUGE UND SVF-ISOLIERUNGSVERFAHREN MIT VERWENDUNG DAVON
CUVE POUR CENTRIFUGEUSE ET PROCÉDÉ D'ISOLATION DE SVF UTILISANT CELLE-CI.

(30) Priority: 07.06.2016 KR 20160070331; 06.10.2016 KR 20160129153
(43) Date of publication of application: 10.04.2019
(73) Proprietor: CG Bio Co., Ltd., Seongnam-si, Gyeonggi-do 13211 (KR)
(72) Inventor: PARK, Jong Ha, Seongnam-si Gyeonggi-do 13208 (KR); HONG, Soon Gee, Seongnam-si Gyeonggi-do 13245 (KR); YOO, Bo Yung, Seoul 05230 (KR); SEO, Jun Hyuk, Hanam-si Gyeonggi-do 13014 (KR); RYU, Hyun Seung, Yongin-si Gyeonggi-do 16894 (KR); KIM, Yong Su, Anyang-si Gyeonggi-do 13921 (KR); KIM, In Ho, Pyeongtaek-si Gyeonggi-do 17809 (KR)
(74) Representative: SONN Patentanwälte OG
(86) International application number: PCT/KR2016/011685
(87) International publication number: WO 2017/213305

(56) References cited:
- WO-A2-2011/052946
- KR-A- 20110 004 629
- KR-A- 20110 045 479
- KR-A- 20130 068 203
- KR-A- 20140 093 821
- KR-A- 20160 006 076
- KR-A- 20160 006 076
- US-A- 5 976 388

## Description

### TECHNICAL FIELD

The present application claims priority to Korean Patent Application Nos. 10-2016-0070331 and 10-2016-0129153 filed on June 7, 2016 and October 6, 2016 in the Republic of Korea.

The present disclosure relates to a centrifuge vessel, a centrifugation method using the same, and a method for isolating stromal vascular fraction (SVF) using the same.

### BACKGROUND ART

Stromal vascular fraction (SVF) can be separated from adipose tissue for cosmetic or therapeutic use. To separate SVF from adipose tissue, washing solution, enzyme, or the like are introduced as necessary, followed by several centrifugation stages using a centrifuge.

When isolating SVF using a general centrifuge, the following problems arise.

First, the SVF isolation efficiency is low in a centrifuge vessel that adopts the side flow path.

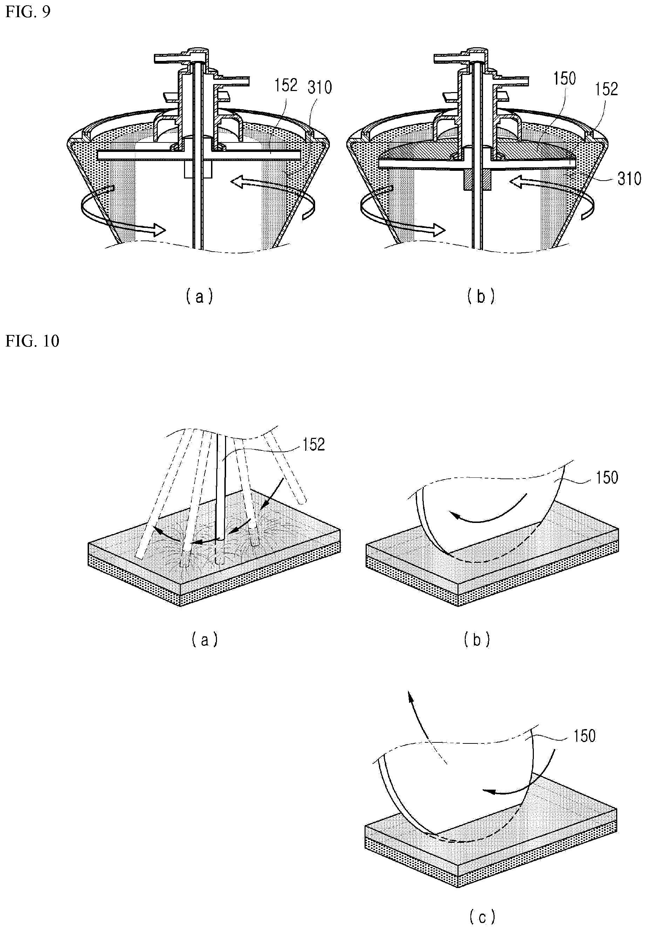

This is due to the fact that the side flow path is not rotated during a rotation of the centrifuge vessel. To specifically describe the SVF isolation process, the liquid mixed with fat, enzyme, and physiological saline is centrifuged by the centrifugal force of the centrifuge, so that the SVF and other substances are separated into respective layers, thus forming a multi-layer structure on a side wall. At this time, the centrifuge vessel and the layers accumulated on the side thereof continue to rotate during centrifugation, while the side flow path connected to the central flow path is not rotated (see FIG. 9a). In view of a plurality of separated layers, the plurality of separated layers are stationary, while the side flow path is rotated (see FIG. 10a). Accordingly, a so-called "stirring effect" is occurred, in which the separated substance layers are stirred by the side flow path, causing a portion of the separated SVF to be re-mixed with the other substances, resulting in a reduction in the SVF separation efficiency.

In other words, the side flow path is necessary to separate the SVF from the adipose tissue through centrifugation and then to discharge the remaining adipose tissue to the outside, but the side flow path also interferes with the SVF isolation.

Second, the yield rate of SVF is low.

As described in Korean Patent No. 10-1316573 and PCT Publication No. WO2008-133874, a robe protruding outward on the side wall or bottom surface of a general centrifuge vessel is present, and the separated substance accumulates in the protruding robe. In these examples, the robe protruding outward of the side wall protrudes only from a part of the entire side wall of the centrifuge vessel. However, since the substance separated by rotation of the centrifuge is generated at all angles in the radial direction, the substance generated at an angle where the protruding robe is not positioned does not converge well to the protruding robe, and accordingly, the yield rate is low. A similar device for SVF separation is also known from PCT Publication No. WO2011-052946.

Third, it is difficult to remove the viscous SVF from the vessel side wall.

SVF has a viscosity. When the SVF is separated by the centrifuge, due to the centrifugal force, the SVF is adhere to the side wall, not the bottom of the centrifuge vessel. A special structure is needed to effectively remove the SVF adhered to the side wall.

To solve these problems, the present applicant has proposed a centrifuge vessel protruding radially at all angles in Korean Patent Publication No. 10-2016-0006076. This provides a flow path with a constant cross-section for the substances therein, and it is possible to remove the SVF precipitated in a flow path with the certain cross-section by exerting a rapid stream thereto through repeated rotation and stop of the vessel.

Fourth, it is not easy to repeatedly use, replace and dispose of the vessel.

The centrifuge vessel in the field of medical technology should be discarded after one use. Therefore, frequent exchanging of the vessel is essential, and it is thus preferable that attaching and detaching the vessel is easy, and the production is simple, and the unit price is low. The complex appearance of the vessel can make installation and removal inconvenient and cause leakage between parts assembled. And it is of course required that the centrifuge maintain excellent tightness when considering the characteristics of the centrifuge.

Other types of centrifuges are known in the field of blood component separation, such as the one disclosed in US patent document US 5,976,388.
(Patent Document 1) KR10-2016-0006076 A1
(Patent Document 2) KR10-1316573 B1
(Patent Document 3) WO2008-133874
(Patent document 4): WO2011-052946
(Patent document 5): US 5,976,388

### DISCLOSURE

### Technical Problem

The present disclosure is designed to solve the problems of the related art.

Specifically, the present disclosure proposes a centrifuge vessel, which is capable of centrifugation through a rotation thereof, removing a certain part during centrifugation, and performing a process of introducing a foreign substance without reducing the SVF isolation efficiency. In particular, the present disclosure intends to propose a centrifuge vessel from which stirring effect is substantially eliminated.

In addition, the present disclosure intends to provide a centrifuge vessel which can increase the yield rate of SVF finally obtained.

Further, the present disclosure intends to provide a centrifuge vessel, which is easily removed after one use and newly mounted, and which has an excellent tightness, low difficulty of manufacture, and low manufacturing cost.

### Technical Solution

According to the invention , there is provided a centrifuge vessel, which includes a body unit 300 that is rotatable, a cover part 200 that covers the body unit 300, and a connection part 100 provided in the cover part 200 and comprising a plurality of connection pipes 111 and 112 in fluid communication with an inside of the body unit 300, in which the body unit 300 includes a side accommodation part 310 formed on a side surface of the body unit 300 by extending at least a part thereof radially outwards, and a plate 150 having a radial end positioned on the side accommodation part 310.

Further, the side accommodation part 310 extends radially in all directions.

The plate 150 is a body of revolution such that the stirring effect does not occur even when the plate 150 is not rotated during rotation of the body unit 300.

Provided is a side flow path 152 in fluid communication with one connection pipe 112 of the plurality of connection pipes 111 and 112 is provided inside the plate 150.

An end of the other connection pipe 111 of the connection pipes 111 and 112 reaches the lower body 303 of the body unit 300.

The connection part 100 further includes a connection pipe housing 115, the other connection pipe 111 of the plurality of connection pipes 111 and 112 reaches the lower body 303 of the body unit 300 through a central flow path 120, a part of the central flow path 120 is provided inside the connection pipe housing 115, the one connection pipe 112 is in fluid communication with the side flow path 152 through a space between the connection pipe housing 115 and the central flow path 120, and a side flow path opening 151 connecting a space between the connection pipe housing 115 and the central flow path 120 to the side flow path 152 is disposed at a center of the plate 150.

The outside of the centrifuge vessel and inside of the side accommodation part 310 are in fluid communication through the connection pipe 112 and the side flow path 152.

A substance accommodated in the side accommodation part 310 is discharged to the outside of the centrifuge vessel through the one connection pipe 112 and the side flow path 152 and a substance outside the centrifuge vessel is introduced into the side accommodating part 310.

The diameter of an upper portion of the connection pipe housing 115 is smaller than a diameter of a lower portion of the connection pipe housing 115, the central flow path 120 is inserted into and coupled with the upper portion of the connection pipe housing 115, and the side flow path opening 151 is inserted into and coupled with the lower portion of the connection pipe housing 115.

It is also preferable that the side accommodation part 310 extends radially outwards of an upper portion, a middle portion, or a lower portion of the body unit 300.

It is also preferable that the body unit 300 includes an upper body 301, a middle body 302, and a lower body 303, a diameter of the upper body 301 gradually increases in an upward direction, and the side accommodating part 310 is a radially outer part of the upper body 301 with an increased diameter.

It is also preferable that the diameter of the lower body 303 gradually decreases in a downward direction, a plurality of stirring members 360 are positioned on an inner surface of the lower body 303, and It is preferable that the plurality of stirring members 360 are arranged to form a plurality of rows oriented toward a center of rotation axis of the body unit 300.

In another aspect of the present disclosure, there is provided a centrifugation method using the centrifuge vessel described above, which includes the steps of separating a substance inside the centrifuge vessel as the body unit 300 is rotated by a rotation of the centrifuge vessel, which is accumulated in the side accommodating part 310, during which the plate 150 is not rotated when the body unit 300 is rotated, thus preventing a stirring effect of the substance that is separated and accumulated in the side accommodating part 310.

In another aspect of the present disclosure, there is provided an SVF isolation method using the centrifugation method as described above, which may include steps of (a) introducing adipose tissue into the body unit 300 through a first connection pipe 111 of the plurality of connection pipes 111 and 112, (b) supplying the washing solution through the first connection pipe 111 and centrifuging by the centrifugation method to separate the adipose tissue into a washed adipose tissue and a contaminant, (c) removing contaminants from the body unit 300 through the first connection pipe 111, (d) supplying an enzyme through the first connection pipe 111, stirring, and centrifuging by the centrifugation method described above to separate the washed adipose tissue into a digested adipose tissue and a primary aqueous solution, (e) removing the digested adipose tissue from the body unit 300 through the second connection pipe 112 of the plurality of connection pipes 111 and 112, (f) separating a primary aqueous solution into SVF and a secondary aqueous solution by supplying a washing solution through the first connection pipe 111, and centrifuging by the centrifugal separation method, wherein the SVF is adhered to the side accommodation part 310, (g) removing the secondary aqueous solution from the body unit 300 through the second connection pipe 112, (h) precipitating the SVF adhered to the side accommodating part 310 by inertia to the lower body 303 by supplying a small amount of washing solution through the first connection pipe 111 and then repeating rotating and stopping the centrifuge vessel, and (i) extracting the SVF from the lower body 303 through the first connection pipe 111.

### Advantageous Effects

The present disclosure gives the following effects. The present disclosure proposes a centrifuge vessel having a plate including a side flow path.

With this configuration, the present disclosure has an advantage that the multi-layered structure provided on the sidewall of the centrifuge vessel is not stirred during the centrifugation, and therefore, stirring effect is substantially eliminated while some of the components precipitated on the side wall can be selectively discharged.

In addition, since the separated SVF is gathered in the side accommodation part positioned radially in all directions, high SVF yield rate is provided.

In addition, the presence of latching jaw or the like allows convenient attachment and detachment, and ease of manufacturing and excellent tightness, and low manufacturing cost are also provided.

Furthermore, as a result of the verification test, it was confirmed that the yield rate and the stability using the centrifuge vessel according to the present disclosure were superior to those of the conventional apparatuses, as shown in FIGS. 13 to 15.

### BRIEF DESCRIPTION OF THE DRAWINGS

The accompanying drawings illustrate a preferred embodiment of the present disclosure and together with the foregoing disclosure, serve to provide further understanding of the technical features of the present disclosure, and thus, the present disclosure is not construed as being limited to the drawing.
FIG. 1 is a perspective view of a centrifuge vessel according to the present disclosure.
FIG. 2 is an exploded perspective view of a centrifuge vessel according to the present disclosure.
FIG. 3 is a cross-sectional view of a centrifuge vessel taken along line A-A' of FIG. 1 according to the present disclosure.
FIG. 4 is a perspective view of a connection part 100 of a centrifuge vessel according to the present disclosure.
FIG. 5 is a perspective view of a part of a connection part 100 of the centrifuge vessel according to the present disclosure.
FIG. 6 is a cross-sectional perspective view of a part of a connection part 100 of a centrifuge vessel according to the present disclosure.
FIGS. 7 and 8 are perspective views of a centrifuge vessel of another embodiment of the present disclosure.
FIGS. 9 and 10 are conceptual views for explaining the characteristics for eliminating the stirring effect of a centrifuge vessel according to the present disclosure.
FIG. 11 is a flowchart for explaining an isolation method of SVF using a centrifuge vessel according to the present disclosure.
FIG. 12 is a schematic view for explaining an isolation method of SVF using a centrifuge vessel according to the present disclosure.
FIGS. 13 to 15 are the results of a verification test of comparing between SVF isolation using a centrifuge vessel according to the present disclosure and SVF isolation using conventional instruments.

### BEST MODE

Hereinafter, a centrifuge vessel according to the present disclosure will be described with reference to the drawings.

### 1. Description of Centrifuge Vessel

The centrifuge vessel according to the present disclosure is mounted in a centrifuge. The centrifuge generally includes a power member that provides power for rotation, a plurality of bags connected to the centrifuge vessel to accommodate the substances to be separated and the additives, and the separated substances or contaminants therein, a control unit for controlling the operation, a display device for outputting the status of operation, and so on. The centrifuge vessel according to the present disclosure can be applied to any kind of centrifuge, and therefore, the centrifuge will not be redundantly described herein.

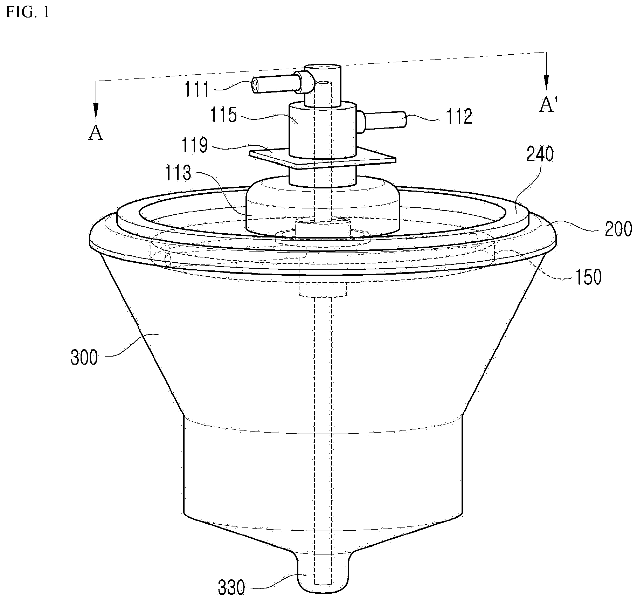

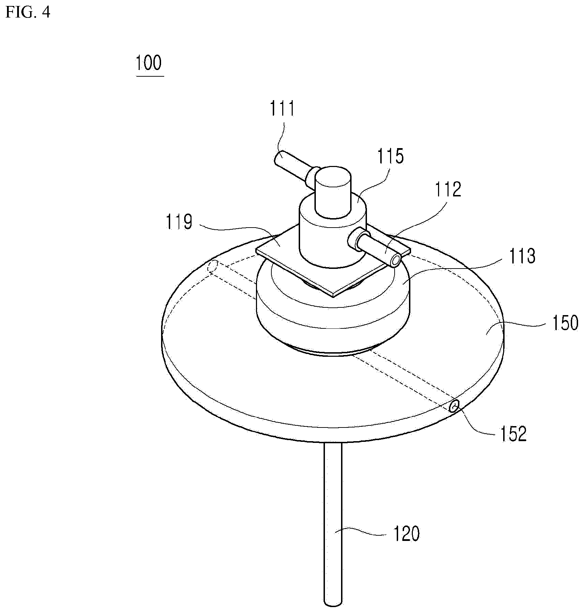

Hereinafter, a centrifuge vessel according to the present disclosure will be described in detail with reference to FIGS. 1 to 6.

### 1.1. Connection part 100

The centrifuge vessel according to the present disclosure includes a connection part 100, a cover part 200, and a body unit 300.

A first connection pipe 111, a second connection pipe 112, a sealing member housing 113, a connection pipe housing 115, and a latching jaw 119 are positioned on an upper portion of the connection part 100. Although the connection part 100 is shown in the drawing as including two connection pipes 111 and 112, two or more connection pipes may be provided as well.

The plurality of connection pipes 111 and 112 provided on the connection part 100 are connected to the bags of the centrifuge by separate tubes or the like. With this configuration, the user may be able to feed the analyte or additive into the centrifuge vessel, or remove the separated substance or contaminants out of the centrifuge vessel.

A plurality of connection pipes 111 and 112 are in fluid communication with a space within the body unit 300, and the flow path configuration will be specifically described below.

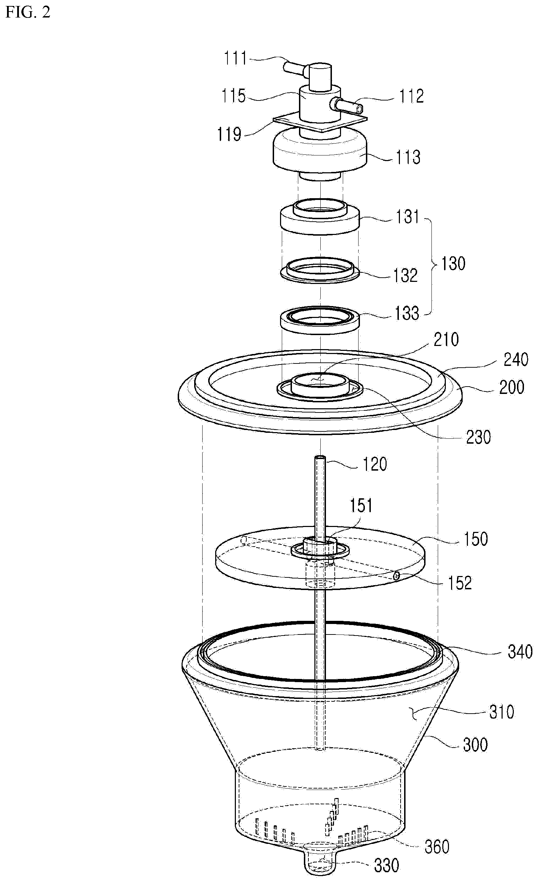

The sealing member housing 113 has a sealing member 130 positioned therein to protect the sealing member 130 from the outside. The sealing member 130 may include a first sealing member 131, a second sealing member 132, and a third sealing member 133 (see FIG. 2), and may be made of an elastic member, a ceramic member, and a magnetic member, respectively, to increase tightness.

The connection pipe housing 115 includes a flow path through which a plurality of connection pipes 111 and 112 are in fluid communication with a space inside the body unit 300, and also protects the flow path from the outside.

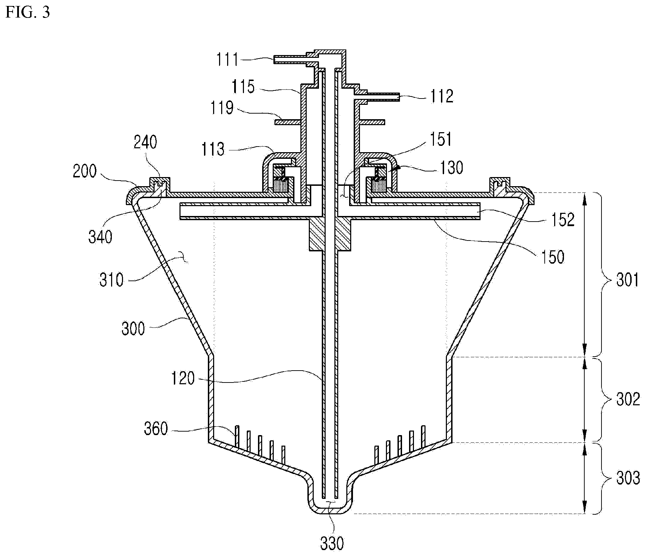

The connection pipe housing 115 is configured such that an upper portion and a lower portion are stepped, and the first connection pipe 111 is connected to the upper portion and the second connection pipe 112 is connected to the lower portion. When the upper and lower portions are stepped, this means that the upper and lower inner diameters of the connection pipe housing 115 are configured differently from each other, which allows the center flow path 120 to be easily inserted into the upper portion, and also allows a protruding portion of the upper plate 150 having the side flow path opening 151 positioned therein to be inserted into the lower portion, and thus allows easy assembly, while maintaining the tightness (see FIG. 3).

The latching jaw 119 refers to a portion where the centrifuge is latched when the centrifuge vessel is mounted on the centrifuge, and it serves to help the centrifuge vessel to be set in position in the centrifuge. When the centrifuge vessel is mounted for use, user can easily mount the centrifuge by latching the centrifuge to the latching jaw 119, and can also easily separate the centrifuge by releasing the latching jaw 119.

The lower portion of the connection part 100 is inserted into the space within the body unit 300 and is sealed from the outside by the cover part 200 and the sealing member 130. A circular plate 150 is positioned under the connection part 100. A radial end of the plate 150 is positioned in the side accommodation part 310. In the drawing, the plate 150 is shown as having a circular plane, that is, the plate 150 itself is in a flat cylindrical shape, but any body of revolution may be used. This will be described in detail below.

A side flow path 152 is provided inside the plate 150 to provide a fluid communication through the second connection pipe 112.

One of the advantages of the plate 150 according to the present disclosure is that the side flow path 152 provided in the plate 150 can be directly connected to the side accommodation part 310. Accordingly, another advantages are obtained such that it is possible to discharge some of the substances sequentially precipitated in the side accommodation part 310 by the centrifugation process through the second connection pipe 112, and in the discharge process and the centrifugation process, the plate 150 as a body of revolution does not cause a stirring effect on the solution collected in the side accommodation part 310, so that the outermost precipitate layer not in contact with the discharge port is not stirred. This will be discussed in detail below with reference to FIGS. 9 and 10.

### 1.2. Cover part 200

The cover part 200 refers to a portion that is seated on the upper portion of the body unit 300.

An opening 210 is positioned at an upper center of the cover part 200. The opening 210 is configured such that the protrusion of the upper plate 150 having the side flow path positioned therein is inserted into and coupled with the opening 210, and the central flow path 120 is passed through the opening 210.

A sealing member seating part 230 is positioned radially outward of the opening 210, and the sealing member 130 is seated thereon.

A body connection part 240 protruding upward is positioned radially outward of the cover part 200. A groove is provided on a lower portion of the body connection part 240 and a cover connection part 340 of the body unit 300 is inserted therein, such that the durability of the centrifuge vessel is increased and separation of the cover part 200 is prevented during rotation.

### 1.3. Body unit 300

The body unit 300 may be divided into an upper body 301, a middle body 302, and a lower body 303 (see FIG. 3).

The side accommodation part 310 is positioned radially outward of the upper body 301. The side accommodation part 310 refers to a portion that protrudes at least radially outward of the body unit 300. During an operation of the centrifuge, substances separated in the centrifuge vessel begin to be accumulated naturally in the side accommodation part 310 by centrifugal force. When the rotation of the centrifuge is completed, substances of low viscosity will descend by gravity and flow to a lower accommodating part 330 of the lower body 303, while substance of high viscosity (e.g., SVF) remain in the side accommodating part 310.

Preferably, the wall of the side accommodation part 310 is radially and outwardly protruded and smoothly connected to the wall of the body unit 300. That is, it is preferable that the upper body 301 and the middle body 302 are connected smoothly. In this case, substances separated in the middle body 302 or the lower body 303 can be naturally delivered to the side accommodation part 310 of the upper body 301. While the diameter of the middle body 302 is shown as being constant in FIG. 3, the diameter may be different as long as the middle body 302 is smoothly connected to the upper body 301.

Further, the side accommodation part 310 extends radially outwards in all directions (that is, by 360 degrees). When only a part of the lobe is extended, it will take the form of the conventional lobe where the SVF is accommodated to some extent. However, when the lobe extends radially outwards in all directions, SVF can be accommodated regardless of directions where the SVF is separated by centrifugation, and as a result, superior yield rate can be expected.

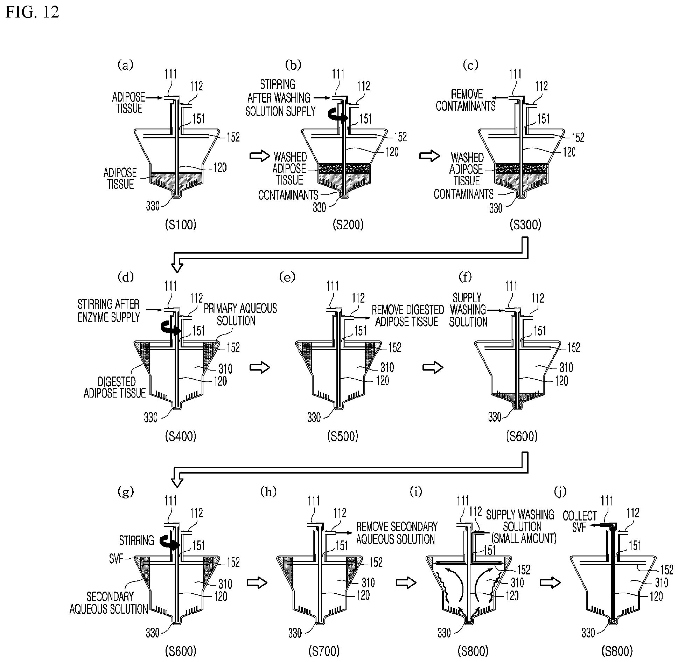

The lower accommodating part 330 is positioned at an end of the lower body 303 and the central flow path 120 is connected to the inside of the lower accommodating part 330. With this configuration, the substances accumulated in the lower accommodating part 330 can be discharged to the outside of the centrifuge vessel (see FIGS. 12c and 12j).

A plurality of stirring members 360 are positioned on an inner surface of the lower body 303, i.e., on a bottom surface of the lower body 303. It is preferable that the plurality of stirring members 360 are arranged so as to form a plurality of rows toward the center of rotation axis of the body unit 300. FIG. 2 illustrates five stirring members 360 in each of three rows, but the number and arrangement may be varied.

Meanwhile, in the illustrated embodiment, the side accommodation part 310 extending radially outward is positioned in the upper body 301, but another embodiment is also possible in which the side accommodation part is positioned in the middle body or the lower body. In the above case, the plate should also be positioned in the middle body or the lower body where the side accommodation part is positioned.

### 1.4. Flow path

The flow path of the centrifuge vessel according to the present disclosure will be described with further reference to FIGS. 3. As used herein, "flow channel" refers to a fluid communication path between the outside of the centrifuge vessel and the inside of the body unit 300.

The present disclosure adopts two independent channels.

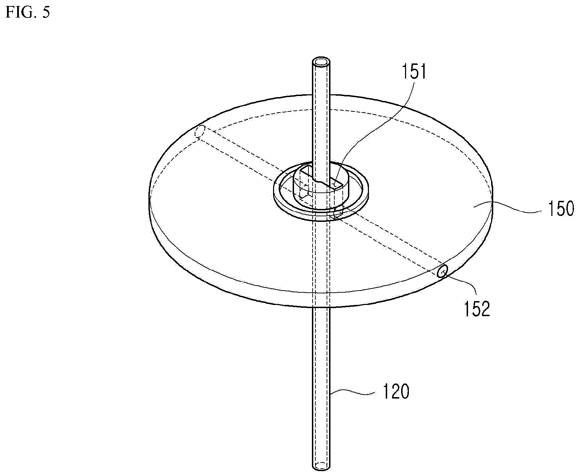

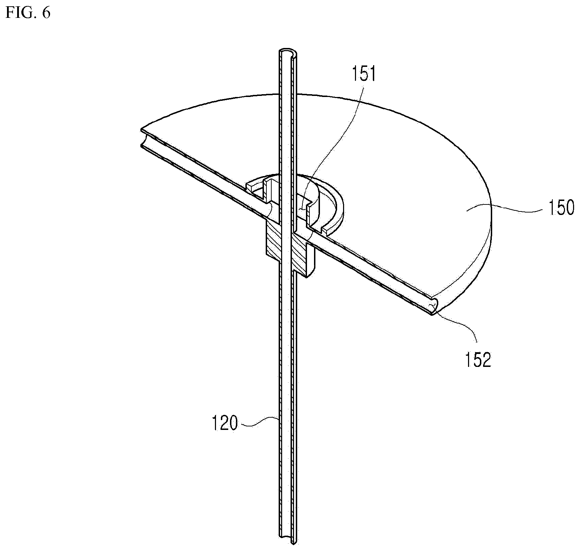

The first flow path includes the first connection pipe 111 and the central flow path 120, and the end thereof is positioned at the lower accommodating part 330. The second flow path includes the second connection pipe 112, a radially outer region of the connection pipe housing 115 (that is, an outer region of the central flow path 120), a side flow path opening 151, and a side flow path 152. An end of the second flow path is positioned in the side accommodation part 310. In this example, the side flow path opening 151 is in a shape that is bent at 90 degrees to connect the second connection pipe 112 and the side flow path 152 (see FIGS. 5 and 6).

The first flow path and the second flow path are independent of each other. In particular, when viewed from the inside of the connection pipe housing 115, the central flow path 120 is positioned nearest to the center, thus constituting the first flow path, and the second flow path is formed on the outside of the wall of the central flow path 120 that serves as a boundary. Accordingly, the two flow paths are independent of each other.

### 2. Description of Assembling Method of Centrifuge Vessel

The centrifuge vessel according to the present disclosure is advantageous in that the vessel is designed to be easily assembled while having an excellent tightness, thus providing low difficulty of manufacturing and low manufacturing cost.

The assembling method will be described with reference to FIG. 2.

The upper portion of the connection part 100, which includes the plurality of connection pipes 111 and 112, the connection pipe housing 115, the latching jaw 119, and the sealing member housing 113, is integrally molded.

The first sealing member 131, the second sealing member 132, and the third sealing member 133 are separately molded due to different substances thereof, and then an upper surface of the second sealing member 132 is inserted into a lower surface of the first sealing member 131 and an upper surface of the third sealing member 133 is inserted into a lower surface of the second sealing member 132 to form an integrated sealing member 130.

The upper surface of the sealing member 130 (that is, the upper surface of the first sealing member 131) is inserted into the inner surface of the sealing member housing 113 and the lower surface of the sealing member 130 (that is, the lower surface of the third sealing member 133) is seated on the sealing member seating part 230 of the cover part 200. In this example, the upper surface of the sealing member 130 is sized and shaped to fit the inner surface of the sealing member housing 113, and the lower surface of the sealing member 130 is sized and shaped to fit the sealing member seating part 230, such that the sealing is maintained.

Next, the lower portion of the connection part 100 including the central flow path 120 and the plate 150 is inserted into and coupled with the assembly of the upper portion of the connection part 100, the sealing member 130 and the cover part 200 from below. Specifically, the central flow path 120 is inserted into the upper portion of the connection pipe housing 115, and the protruding portion of the upper plate 150 where the side flow path opening 151 is positioned is inserted into the lower portion of the connection pipe housing 115 (see FIG. 3).

Next, the body unit 300 is mounted on the cover part 200. That is, the cover connection part 340 of the body unit 300 is inserted into a lower portion of the body connection part 240 of the cover part 200 and fixed in position. A separate adhering or fusing may be further provided for the inserting and fixing.

### 3. Description of Other Embodiments

As shown in FIGS. 1 to 6, the centrifuge vessel according to the embodiment of the present disclosure described above has the side accommodation part 310 positioned in the upper portion of the main body 301.

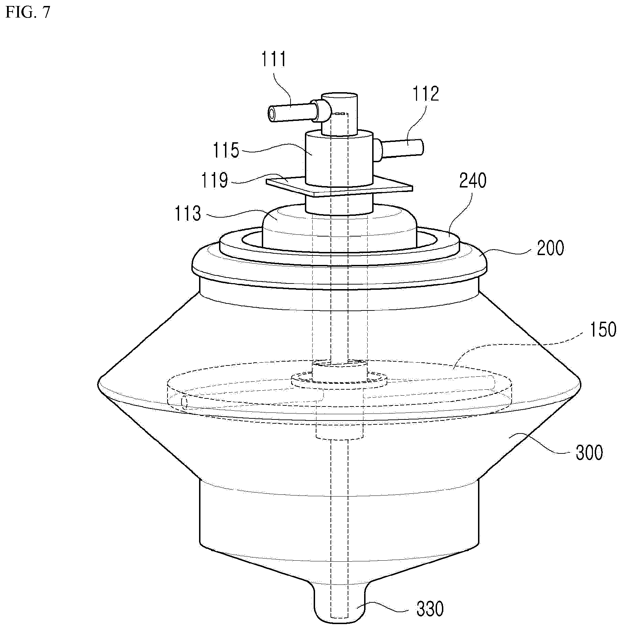

As shown in FIG. 7, a centrifuge vessel according to another embodiment of the present disclosure has the side accommodation part 310 positioned in the middle portion of the main body 302.

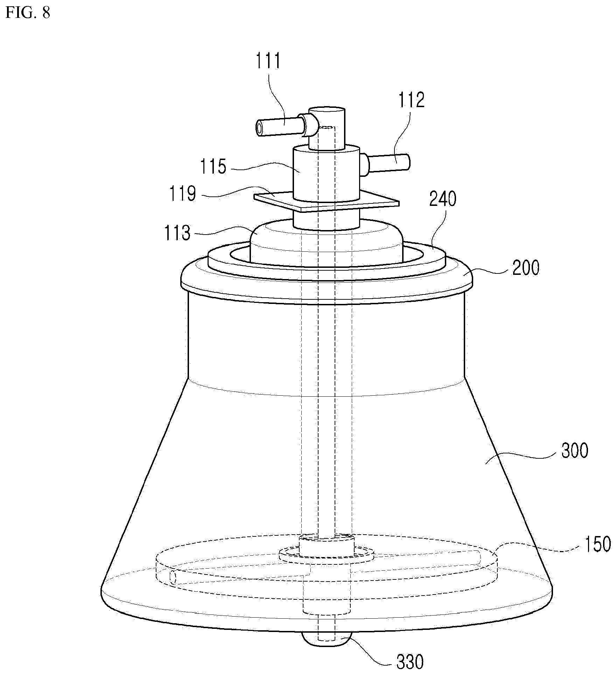

As shown in FIG. 8, a centrifuge vessel according to still another embodiment of the present disclosure has the side accommodation part 310 positioned in the lower portion of the main body 303.

In all of the three embodiments shown in FIGS. 1, 7 and 8, the side accommodation part 310 has at least a part extending radially outwards, or preferably, the side accommodation part 310 extends radially outwards in all directions. Further, in all three embodiments, an end of the plate 150 is positioned in the side accommodation part 310.

### 4. Substantial Elimination of the Stirring Effect

The characteristic of a centrifuge vessel of eliminating the stirring effect according to the present disclosure will be described below with reference to FIGS. 9 and 10. FIGS. 9 and 10a illustrate the stirring effect shown in the related art (or in particular, in Korean Patent Publication No. 10-2016-0006076), and FIG. 10b illustrates an example of the present disclosure.

In a centrifuge vessel according to the present disclosure, the substance to be separated in the cover part 200, the body unit 300 and the body unit 300 are rotated as the centrifuge is operated. In this situation, the components included in the connection part 100 are not rotated. That is, the central flow path 120, the plate 150, and the side flow path 152 inside the plate 150 included in the connection part 100 are not rotated. This is because the connection part 100 is connected to a substance accommodating bag or the like of the centrifuge that supplies the substance through a tube (not shown) or the like.

This is also the case of the related art in which the plate 150 is not provided, and accordingly, as shown in FIG. 9a, the body unit 300 and the substance to be separated included therein are rotated, but the side flow path 152 is not rotated when the centrifuge is operated. When the process proceeds to some extent, the substances to be separated are separated into SVF or the like to form a multi-layered structure, and the substances in the multi-layered structure are rotated, but the side flow path 152 is not rotated.

As shown in FIG. 10a, in a relative observation of this from the perspective of the multiple layers separated, it means that only the side flow path 152 is rotated, while the multiple layers are in a stationary state. Accordingly, the side flow path 152 stirs and disturbs the multiple layers as it is repeatedly dipped into and removed from the multiple separated layers. This causes some of the separated substances including SVF to be re-mixed, which lowers the isolation efficiency.

However, in the embodiment of the present disclosure provided with the circular plate 150, different aspects are provided.

As shown in FIG. 10b, in a relative observation from the side of the multiple layers separated, only the plate 150 is rotated, while the multiple layers are in a stationary state. However, only the plate 150 is rotated due to its circular shape, in the same three-dimensional position. That is, the plate is not newly dipped or removed. Accordingly, the multiple layers are not stirred or disturbed, and the stirring effect in which the separated substances including the SVF are re-mixed again is substantially eliminated.

Meanwhile, the plate 150 for preventing the stirring effect does not necessarily have to be circular, and any shape may be used as long as it is a shape of a body of revolution. As used herein, the "body of revolution" means a figure that always has a constant shape on the cross-section including the rotation axis.

For example, while the plate 150 is circular in FIG. 10b, the cross-section from the side of the multiple layers is constant as a square.

The plate 150 may be a body of revolution having a triangular cross-section rather than a rectangular shape. In this case, the plate 150 itself may have a flat conical shape, and the stirring effect is still not occurred, which is preferable.

The plate 150 may be a body of revolution having a parallelepipedal cross-section. In this case, the plate 150 itself will be of an umbrella shape, and again, the stirring effect is not occurred, which is preferable.

### 5. Description of SVF Isolation Method Using Centrifuge Vessel

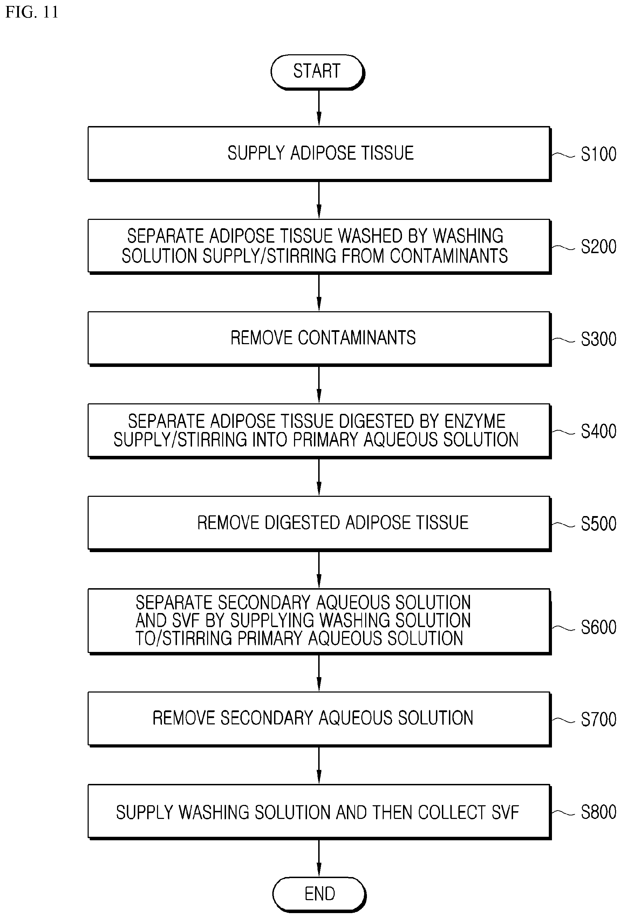

An SVF isolation method using a centrifuge vessel according to the present disclosure will be described below with reference to FIGS. 11 and 12.

FIGS. 12a to 12j show the respective steps described with reference to FIG. 11, in which corresponding reference numerals (S100 to S800) are added at the bottom of each drawing.

The adipose tissue is supplied into the body unit 300 through the first connection pipe 111 (S100).

Next, washing solution is supplied into the body unit 300 through the first connection pipe 111, and then the centrifuge is operated, rotating and stirring for a predetermined time (S200). Upon completion of the rotating and stirring, the washed adipose tissue and contaminants including blood or the like are separated into upper and lower layers, respectively. The lower layer of the separated contaminants are discharged to the exterior through the central flow path 120 whose end is positioned in the lower accommodating part 330, and removed (S300).

Next, an enzyme such as collagenase for example is supplied and the centrifuge is operated to rotate and stir for a predetermined time (S400). Upon completion of rotating and stirring, the adipose tissue and primary aqueous solution digested by the enzyme are separated radially inwards and outwards, respectively. The digested adipose tissues that are separated radially inwards are discharged to the outside through the side flow path 152 whose end is positioned in the side accommodation part 310, and removed (S500).

Then the washing solution is additionally supplied to the remaining primary aqueous solution, after which rotating and stirring are performed for a predetermined time (S600). Thus, the secondary aqueous solution and SVF are separated radially inwards and outwards, respectively. The secondary aqueous solution that is separated radially inwards is discharged to the outside through the side flow path 152 whose end is positioned in the side accommodation part 310, and removed (S700).

Accordingly, only the SVF remains in the side accommodation part 310, and it is remained positioned in the side accommodation part 310 due to the viscosity of the SVF, that is, the SVF does not descend downward even with its own weight. Accordingly, after a small amount of washing solution is supplied through the first connection pipe 111, the centrifuge vessel repeats rotating and stopping. Because the centrifugal force from the rotation causes the washing solution to be spread widely in the side accommodation part 310, by the repeated rotation and stopping, the SVF is precipitated in the lower body 303 by the inertia imparted to the washing solution. Next, the SVF precipitated in the lower body 303 is extracted and collected through the first connection pipe 111 (S800).

### 6. Verification Test

Tests were conducted to verify the performance of the centrifuge vessel according to the present disclosure.

The fat which was aspirated from the abdomen and thigh of 10 male and female adults between the ages of 20 and 40 was introduced into a centrifuge vessel according to the present disclosure, and then SVF was separated according to the method described with reference to FIGS. 11 and 12. The tests were conducted with first group introduced with 50 ml and the second group introduced with 100 ml. As an enzyme, 50 mg of collagenase was diluted in 50 ml of Hartmann's solution, compared to 50 ml of fat.

The number of obtained cells per 1g of introduced fat (Cells/g), the cell viability (%), and the collagenase activity (PZU/ml), that is, the residual amount of collagenase, were calculated based on the final product obtained according to the method described in FIGS. 11 and 12. Cell counting for calculating the number of obtained cells and viability was performed using an automated cell counter.

Among the conventional instruments currently available in the market, Multi Station^{©}, Cha-Station^{©}, Lipokitⓒ, and Celution^{©} were selected. The number of obtained cells per 1 g of introduced fat (Cells/g), the cell viability (%), and the collagenase activity (PZU/ml) of each of the instruments are already well known, and accordingly, the corresponding information was compared with the results according to the present disclosure.

### (1) Number of Obtained Cells

As shown in FIG. 13, it was observed that when the centrifuge vessel according to the present disclosure was used, 2 to 50 times or more cells were obtained as compared to the results obtained from Multi Station^{©}, Cha-Station^{©}, and Lipokitⓒ. However, the number of obtained cells was similar to the results obtained from Celution^{©}.

### (2) Cell Viability

As shown in FIG. 14, the viability of the centrifuge vessel according to the present disclosure was very excellent as compared to the results obtained from Lipokit^{©} and Celution^{©}. However, the cell viability of the centrifuge vessel according to the present disclosure was similar or somewhat lower when compared to the results obtained from Multi Station^{©} and Cha-Station^{©}.

Then the number of obtained cells was compared with the following results.

Compared with the present disclosure, Multi Station^{©} of the related art exhibited half of the number of obtained cells or less, but the cell viability was somewhat higher. Compared with the present disclosure, Lipokit^{©} and Celution^{©} exhibited similar number of obtained cells, but lower cell viabilitys. That is, the related art exhibited a low viability when a large amount of cells are obtained, and the number of obtained cells was low when the viability was high.

Therefore, it was confirmed that the present disclosure has excellent cell viability as well as the number of cells obtained, and thus has very excellent final yield rate as compared with the related art.

### (3) Enzyme Residue

As a result of the collagenase activity as shown in FIG. 15, when the centrifuge vessel according to the present disclosure was used, the amount of collagenase residue was about 20% or less of the average value in Multi Station^{©}, Cha-Station^{©}, Lipokitⓒ, and Celution^{©}, thus it was confirmed that it is more stable.

It will be apparent to those skilled in the art that various modifications and variations can be made to the present disclosure without departing scope of the present disclosure as defined by the following claims and their equivalents. Therefore, the scope of protection of present disclosure is determined by the claims.

## Claims

1. A centrifuge vessel comprising:
a body unit (300) that is rotatable;
a cover part (200) that covers the body unit (300); and
a connection part (100) provided in the cover part (200) and comprising a plurality of connection pipes (111) and (112) in fluid communication with an inside of the body unit (300),
wherein the body unit (300) comprises
a side accommodation part (310) formed on a side surface of the body unit (300) by extending radially outwards in all directions and protruding radially outward of the body unit (300),
**characterized in that**
the centrifugation vessel comprises a plate (150), said plate (150) being a body of revolution and having a radial end positioned on the side accommodation part (310),
wherein a side flow path (152) in fluid communication with one connection pipe (112) of the plurality of connection pipes (111, 112) is provided inside the plate (150),
wherein the plate (150) is configured not to rotate when the body unit (300) rotates,
wherein an end of the other connection pipe (111) of the connection pipes (111) and (112) reaches the lower body (303) of the body unit (300),
wherein the connection part (100) further comprises a connection pipe housing (115),
the other connection pipe (111) of the plurality of connection pipes (111) and (112) reaches the lower body (303) of the body unit (300) through a central flow path (120),
a part of the central flow path (120) is provided inside the connection pipe housing (115),
the one connection pipe (112) is in fluid communication with the side flow path (152) through a space between the connection pipe housing (115) and the central flow path (120), and
a side flow path opening (151) connecting a space between the connection pipe housing (115) and the central flow path (120) to the side flow path (152) is disposed at a center of the plate (150),
wherein outside of the centrifuge vessel and inside of the side accommodation part (310) are in fluid communication through the connection pipe (112) and the side flow path (152),
wherein a substance accommodated in the side accommodation part (310) is discharged to the outside of the centrifuge vessel through the one connection pipe (112) and the side flow path (152), and a substance outside the centrifuge vessel is introduced into the side accommodating part (310),
wherein a diameter of an upper portion of the connection pipe housing (115) is smaller than a diameter of a lower portion of the connection pipe housing (115),
the central flow path (120) is inserted into and coupled with the upper portion of the connection pipe housing (115), and
the side flow path opening (151) is inserted into and coupled with the lower portion of the connection pipe housing (115).

2. The centrifuge vessel according to claim 1, wherein the side accommodation part (310) extends radially outwards of an upper portion, a middle portion, or a lower portion of the body unit (300).

3. The centrifuge vessel according to claim 2, wherein the body unit (300) comprises an upper body (301), a middle body (302), and a lower body (303),
a diameter of the upper body (301) gradually increases in an upward direction, and
the side accommodating part (310) is a radially outer part of the upper body (301) with an increased diameter.

4. The centrifuge vessel according to claim 3, wherein the diameter of the lower body (303) gradually decreases in a downward direction,
a plurality of stirring members (360) are positioned on an inner surface of the lower body (303), and
the plurality of stirring members (360) are arranged to form a plurality of rows oriented toward a center of rotation axis of the body unit (300).

5. A centrifugation method using the centrifuge vessel according to claim 1, comprising steps of:
separating a substance inside the centrifuge vessel as the body unit (300) is rotated by a rotation of the centrifuge vessel, which is accumulated in the side accommodating part (310), during which the plate (150) is not rotated when the body unit (300) is rotated, thus preventing a stirring effect of the substance that is separated and accumulated in the side accommodating part (310).

6. An stromal vascular fraction (SVF) isolation method using the centrifugation method according to claim 5, comprising steps of:
(a) introducing adipose tissue into the body unit (300) through a first connection pipe (111) of the plurality of connection pipes (111) and (112);
(b) supplying the washing solution through the first connection pipe (111) and centrifuging by the centrifugation method to separate the adipose tissue into a washed adipose tissue and a contaminant;
(c) removing contaminants from the body unit (300) through the first connection pipe (111);
(d) supplying an enzyme through the first connection pipe (111) and centrifuging by the centrifugation method to separate the washed adipose tissue into a digested adipose tissue and a primary aqueous solution;
(e) removing the digested adipose tissue from the body unit (300) through the second connection pipe (112) of the plurality of connection pipes (111) and (112);
(f) separating a primary aqueous solution into SVF and a secondary aqueous solution by supplying a washing solution through the first connection pipe (111), stirring, and centrifuging by the centrifugal separation method, wherein the SVF is adhered to the side accommodation part (310);
(g) removing the secondary aqueous solution from the body unit (300) through the second connection pipe (112);
(h) precipitating the SVF adhered to the side accommodating part (310) by inertia to the lower body (303) by supplying a small amount of washing solution through the first connection pipe (111) and then repeating rotating and stopping the centrifuge vessel; and
(i) extracting the SVF precipitated in the lower body (303) through the first connection pipe (111).

## Patentansprüche

1. Zentrifugengefäß, aufweisend:
eine Körpereinheit (300), die drehbar ist;
ein Abdeckteil (200), das die Körpereinheit (300) abdeckt;
und
ein Verbindungsteil (100), das in dem Abdeckteil (200) bereitgestellt ist und mehrere Verbindungsleitungen (111) und (112) in Fluidverbindung mit einer Innenseite der Körpereinheit (300) aufweist,
wobei die Körpereinheit (300) Folgendes aufweist
einen seitlichen Aufnahmeteil (310), der an einer Seitenfläche der Körpereinheit (300) ausgebildet ist, indem er sich radial nach außen in alle Richtungen erstreckt und radial nach außen von der Körpereinheit (300) vorsteht,
**dadurch gekennzeichnet, dass**
das Zentrifugengefäß eine Platte (150) aufweist, wobei die Platte (150) ein Rotationskörper ist und ein radiales Ende hat, das an dem seitlichen Aufnahmeteil (310) positioniert ist,
wobei ein seitlicher Strömungsweg (152) in Fluidverbindung mit einer Verbindungsleitung (112) der mehreren Verbindungsleitungen (111, 112) innerhalb der Platte (150) bereitgestellt ist,
wobei die Platte (150) dazu konfiguriert ist, sich nicht zu drehen, wenn sich die Körpereinheit (300) dreht,
wobei ein Ende der anderen Verbindungsleitung (111) der Verbindungsleitungen (111) und (112) den unteren Körper (303) der Körpereinheit (300) erreicht,
wobei das Verbindungsteil (100) ferner ein Verbindungleitungsgehäuse (115) aufweist,
wobei die andere Verbindungsleitung (111) der mehreren Verbindungsleitungen (111) und (112) den unteren Körper (303) der Körpereinheit (300) durch einen zentralen Strömungsweg (120) erreicht,
ein Teil des zentralen Strömungswegs (120) innerhalb des Verbindungsleitungsgehäuses (115) bereitgestellt ist,
die eine Verbindungsleitung (112) durch einen Raum zwischen dem Verbindungleitungsgehäuse (115) und dem zentralen Strömungsweg (120) in Fluidverbindung mit dem seitlichen Strömungsweg (152) steht, und
eine seitliche Strömungswegöffnung (151), die einen Raum zwischen dem Verbindungleitungsgehäuse (115) und dem zentralen Strömungsweg (120) mit dem seitlichen Strömungsweg (152) verbindet, in einer Mitte der Platte (150) angeordnet ist,
wobei die Außenseite des Zentrifugengefäßes und die Innenseite des seitlichen Aufnahmeteils (310) durch die Verbindungsleitung (112) und den seitlichen Strömungsweg (152) in Fluidverbindung stehen,
wobei eine in dem seitlichen Aufnahmeteil (310) aufgenommene Substanz durch die eine Verbindungsleitung (112) und den seitlichen Strömungsweg (152) zur Außenseite des Zentrifugengefäßes abgeleitet wird und eine Substanz außerhalb des Zentrifugengefäßes in den seitlichen Aufnahmeteil (310) eingeführt wird,
wobei ein Durchmesser eines oberen Abschnitts des Verbindungleitungsgehäuses (115) kleiner als ein Durchmesser eines unteren Abschnitts des Verbindungleitungsgehäuses (115) ist,
der zentrale Strömungsweg (120) in den oberen Abschnitt des Verbindungleitungsgehäuses (115) eingesetzt und damit gekoppelt wird und
die seitliche Strömungswegöffnung (151) in den unteren Abschnitt des Verbindungleitungsgehäuses (115) eingesetzt und damit gekoppelt wird.

2. Zentrifugengefäß nach Anspruch 1, wobei der seitliche Aufnahmeteil (310) sich radial nach außen von einem oberen Abschnitt, einem mittleren Abschnitt oder einem unteren Abschnitt der Körpereinheit (300) erstreckt.

3. Zentrifugengefäß nach Anspruch 2, wobei die Körpereinheit (300) einen oberen Körper (301), einen mittleren Körper (302) und einen unteren Körper (303) aufweist,
wobei ein Durchmesser des oberen Körpers (301) allmählich in einer Aufwärtsrichtung zunimmt und
der seitliche Aufnahmeteil (310) ein radial äußerer Teil des oberen Körpers (301) mit einem vergrößerten Durchmesser ist.

4. Zentrifugengefäß nach Anspruch 3, wobei der Durchmesser des unteren Körpers (303) in einer Abwärtsrichtung allmählich abnimmt,
wobei mehrere Rührelemente (360) an einer Innenfläche des unteren Körpers (303) positioniert sind und
die mehreren Rührelemente (360) angeordnet sind, um mehrere Reihen zu bilden, die in Richtung einer Mitte der Drehachse der Körpereinheit (300) ausgerichtet sind.

5. Zentrifugierverfahren unter Verwendung des Zentrifugengefäßes nach Anspruch 1, welches die folgenden Schritte aufweist:
Trennen einer in dem seitlichen Aufnahmeteil (310) angesammelten Substanz innerhalb des Zentrifugengefäßes, wobei die Körpereinheit (300) durch eine Drehung des Zentrifugengefäßes gedreht wird, wobei die Platte (150) nicht gedreht wird, während die Körpereinheit (300) gedreht wird, wodurch ein Rühreffekt der Substanz, die in dem seitlichen Aufnahmeteil (310) getrennt und angesammelt ist, verhindert wird.

6. Verfahren zur Isolierung der stromal-vaskulären Fraktion (SVF) unter Verwendung des Zentrifugierverfahrens nach Anspruch 5, das die folgenden Schritte aufweist:
(a) Einführen von Fettgewebe in die Körpereinheit (300) durch eine erste Verbindungsleitung (111) der mehreren Verbindungsleitungen (111) und (112);
(b) Zuführen der Waschlösung durch die erste Verbindungsleitung (111) und Zentrifugieren durch das Zentrifugierverfahren, um das Fettgewebe in ein gewaschenes Fettgewebe und eine Verunreinigung zu trennen;
(c) Entfernen von Verunreinigungen aus der Körpereinheit (300) durch die erste Verbindungsleitung (111);
(d) Zuführen eines Enzyms durch die erste Verbindungsleitung (111) und Zentrifugieren durch das Zentrifugierverfahren, um das gewaschene Fettgewebe in ein verdautes Fettgewebe und eine primäre wässrige Lösung zu trennen;
(e) Entfernen des verdauten Fettgewebes aus der Körpereinheit (300) durch die zweite Verbindungsleitung (112) der mehreren Verbindungsleitungen (111) und (112);
(f) Trennen einer primären wässrigen Lösung in SVF und eine sekundäre wässrige Lösung durch Zuführen einer Waschlösung durch die erste Verbindungsleitung (111), Rühren und Zentrifugieren durch das Zentrifugierverfahren, wobei die SVF an dem seitlichen Aufnahmeteil (310) haftet;
(g) Entfernen der sekundären wässrigen Lösung aus der Körpereinheit (300) durch die zweite Verbindungsleitung (112);
(h) Ausfällen der SVF, die durch Trägheit an dem seitlichen Aufnahmeteil (310) haftet, in den unteren Körper (303) durch Zuführen einer kleinen Menge an Waschlösung durch die erste Verbindungsleitung (111) und dann Wiederholen des Drehens und Anhaltens des Zentrifugengefäßes und
(i) Extrahieren der im unteren Körper (303) ausgefällten SVF durch die erste Verbindungsleitung (111).

## Revendications

1. Récipient de centrifugeuse comprenant:
une unité formant corps (300) qui est rotative;
une partie formant couvercle (200) qui recouvre l'unité formant corps (300); et
une partie de liaison (100) prévue dans la partie formant couvercle (200) et comprenant une pluralité de tubes de liaison (111) et (112) en communication fluidique avec un intérieur de l'unité formant corps (300),
où l'unité formant corps (300) comprend
une partie de logement latérale (310) formée sur une surface latérale de l'unité formant corps (300) en s'étendant radialement vers l'extérieur dans toutes les directions et en faisant saillie radialement vers l'extérieur de l'unité formant corps (300),
**caractérisé en ce que**
le récipient de centrifugation comprend une plaque (150), ladite plaque (150) étant un corps de révolution et ayant une extrémité radiale positionnée sur la partie de logement latérale (310),
où un passage d'écoulement latéral (152) en communication fluidique avec un tube de liaison (112) de la pluralité de tubes de liaison (111, 112) est prévu à l'intérieur de la plaque (150),
où la plaque (150) est configurée pour ne pas tourner lorsque l'unité formant corps (300) tourne,
où une extrémité de l'autre tube de liaison (111) des tubes de liaison (111) et (112) atteint le corps inférieur (303) de l'unité formant corps (300),
où la partie de liaison (100) comprend en outre un boîtier de tubes de liaison (115),
l'autre tube de liaison (111) de la pluralité de tubes de liaison (111) et (112) atteint le corps inférieur (303) de l'unité formant corps (300) par un passage d'écoulement central (120),
une partie du passage d'écoulement central (120) est prévue à l'intérieur du boîtier de tubes de liaison (115),
le tube de liaison (112) est en communication fluidique avec le passage d'écoulement latéral (152) par un espace entre le boîtier de tubes de liaison (115) et le passage d'écoulement central (120), et
une ouverture de passage d'écoulement latéral (151) reliant un espace entre le boîtier de tubes de liaison (115) et le passage d'écoulement central (120) au passage d'écoulement latéral (152) est disposée au niveau d'un centre de la plaque (150),
où l'extérieur du récipient de centrifugeuse et l'intérieur de la partie de logement latérale (310) sont en communication fluidique par le tube de liaison (112) et le passage d'écoulement latéral (152),
où une substance logée dans la partie de logement latérale (310) est évacuée à l'extérieur du récipient de centrifugeuse par le tube de liaison (112) et le passage d'écoulement latéral (152), et une substance à l'extérieur du récipient de centrifugeuse est introduite dans la partie de logement latérale (310),
où un diamètre d'une partie supérieure du boîtier de tubes de liaison (115) est plus petit qu'un diamètre d'une partie inférieure du boîtier de tubes de liaison (115),
le passage d'écoulement central (120) est inséré dans et couplé à la partie supérieure du boîtier de tubes de liaison (115), et
l'ouverture de passage d'écoulement latéral (151) est insérée dans et couplée à la partie inférieure du boîtier de tubes de liaison (115).

2. Récipient de centrifugeuse selon la revendication 1, où la partie de logement latérale (310) s'étend radialement vers l'extérieur d'une partie supérieure, d'une partie intermédiaire ou d'une partie inférieure de l'unité formant corps (300).

3. Récipient de centrifugeuse selon la revendication 2, où l'unité formant corps (300) comprend un corps supérieur (301), un corps intermédiaire (302) et un corps inférieur (303),
un diamètre du corps supérieur (301) augmente progressivement dans une direction vers le haut, et
la partie de logement latérale (310) est une partie radialement externe du corps supérieur (301) avec un diamètre accru.

4. Récipient de centrifugeuse selon la revendication 3, où le diamètre du corps inférieur (303) diminue progressivement dans une direction vers le bas,
une pluralité d'éléments d'agitation (360) sont positionnés sur une surface intérieure du corps inférieur (303), et
la pluralité d'éléments d'agitation (360) sont agencés pour former une pluralité de rangées orientées vers un centre d'axe de rotation de l'unité formant corps (300).

5. Procédé de centrifugation utilisant le récipient de centrifugeuse selon la revendication 1, comprenant les étapes de:
séparation d'une substance à l'intérieur du récipient de centrifugeuse lorsque l'unité formant corps (300) est entraînée en rotation par une rotation du récipient de centrifugeuse, qui est accumulée dans la partie de logement latérale (310), pendant laquelle la plaque (150) n'est pas entraînée en rotation lorsque l'unité formant corps (300) est entraînée en rotation, empêchant ainsi un effet d'agitation de la substance qui est séparée et accumulée dans la partie de logement latérale (310).

6. Procédé d'isolement de la fraction vasculaire stromale (FVS) utilisant le procédé de centrifugation selon la revendication 5, comprenant les étapes de:
(a) introduction de tissu adipeux dans l'unité formant corps (300) par un premier tube de liaison (111) de la pluralité de tubes de liaison (111) et (112);
(b) apport de la solution de lavage par le premier tube de liaison (111) et centrifugation par le procédé de centrifugation pour séparer le tissu adipeux en un tissu adipeux lavé et un contaminant;
(c) retrait des contaminants de l'unité formant corps (300) par le premier tube de liaison (111);
(d) apport d'une enzyme par le premier tube de liaison (111) et centrifugation par le procédé de centrifugation pour séparer le tissu adipeux lavé en un tissu adipeux digéré et une solution aqueuse primaire;
(e) retrait du tissu adipeux digéré de l'unité formant corps (300) par le second tube de liaison (112) de la pluralité de tubes de liaison (111) et (112);
(f) séparation d'une solution aqueuse primaire en FVS et une solution aqueuse secondaire par apport d'une solution de lavage par le premier tube de liaison (111), agitation et centrifugation par le procédé de séparation centrifuge, où la FVS adhère à la partie de logement latérale (310);
(g) retrait de la solution aqueuse secondaire de l'unité formant corps (300) par le second tube de liaison (112);
(h) précipitation de la FVS adhérant à la partie de logement latérale (310) par inertie sur le corps inférieur (303) par apport d'une petite quantité de solution de lavage par le premier tube de liaison (111) puis répétition de la rotation et de l'arrêt du récipient de centrifugeuse; et
(i) extraction de la FVS précipitée dans le corps inférieur (303) par le premier tube de liaison (111).
